# FASCICULE DE BREVET EUROPEEN

(11) **EP 2 607 354 B1**
(45) Date de publication et mention de la délivrance du brevet: **16.07.2014**
(21) Numéro de dépôt: 12197929.8
(22) Date de dépôt: 19.12.2012
(51) Int. Cl.: C07D 223/16

(54) **NOUVEAU PROCEDE DE SYNTHESE DE L'IVABRADINE ET DE SES SELS D'ADDITION A UN ACIDE PHARMACEUTIQUEMENT ACCEPTABLE**
VERFAHREN ZUR HERSTELLUNG VON IVABRADINE UND DEREN PHARMAZEUTISCH VERTRÄGLICHE SÄUREADDITIONSSALZE
PROCESS FOR THE SYNTHESIS OF IVABRADINE AND ADDITION SALTS THEREOF AND ITS PHARMACEUTICALLY ACCEPTABLE ACID

(30) Priorité: 20.12.2011 FR 1103934
(43) Date de publication de la demande: 26.06.2013
(73) Titulaire: Les Laboratoires Servier, 92284 Suresnes Cedex (FR)
(72) Inventeur: Renaud, Jean-Luc, 14112 Bieville-Beuville (FR); Pannetier, Nicolas, 14000 Caen (FR); Gaillard, Sylvain, 14000 Caen (FR); Lecouve, Jean-Pierre, 76600 Le Havre (FR); Vaysse-Ludot, Lucile, 76490 Saint-Wandrille-Rancon (FR)

(56) Documents cités:
- EP-A1- 2 036 892
- EP-A1- 2 202 225
- WO-A1-2005/110993
- R.CARLSON, T.LEJON, T.LUNDSTEDT AND E. LE CLOUEREC: "An Optimized Procedure for the Reductive Amination of Acetophenone by the Leuckart Reaction", ACTA CHEMICA SCANDINAVICA, vol. 47, 1993, pages 1046-1049, XP002671634, ISSN: 0904-213X

## Description

La présente invention concerne un procédé de synthèse de l'ivabradine de formule (I) : 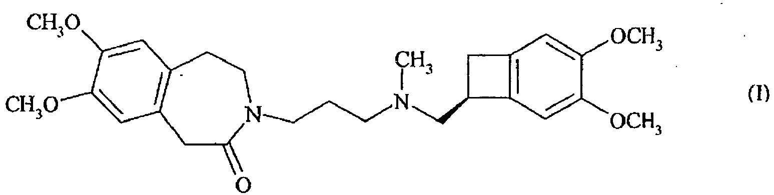 ou 3-{3-[{[(7S)-3,4-diméthoxybicyclo[4.2.0]octa-1,3,5-trién-7-yl]méthyl}(méthyl)amino] propyl}-7,8-diméthoxy-1,3,4,5-tétrahydro-2*H*-3-benzazépin-2-one,
de ses sels d'addition à un acide pharmaceutiquement acceptable et de leurs hydrates.

L'ivabradine, ainsi que ses sels d'addition à un acide pharmaceutiquement acceptable, et plus particulièrement son chlorhydrate, possèdent des propriétés pharmacologiques et thérapeutiques très intéressantes, notamment des propriétés bradycardisantes, qui rendent ces composés utiles dans le traitement ou la prévention des différentes situations cliniques d'ischémie myocardique telles que l'angine de poitrine, l'infarctus du myocarde et les troubles du rythme associés, ainsi que dans les différentes pathologies comportant des troubles du rythme, notamment supra-ventriculaires, et dans l'insuffisance cardiaque.

La préparation et l'utilisation en thérapeutique de l'ivabradine et de ses sels d'addition à un acide pharmaceutiquement acceptable, et plus particulièrement de son chlorhydrate, ont été décrites dans le brevet européen EP 0 534 859. Malheureusement, la voie de synthèse de l'ivabradine décrite dans ce brevet ne conduit au produit attendu qu'avec un rendement de 1%.

Une autre voie de synthèse de l'ivabradine, basée sur une réaction d'amination réductrice, a été décrite dans le brevet européen EP 1 589 005.

L'amination réductrice est une voie d'accès privilégiée pour préparer des amines. Dans la mesure où elle ne requiert pas d'isoler l'imine intermédiaire formée, cette réaction de couplage entre un aldéhyde et une amine en présence d'un agent de réduction est largement utilisée pour la synthèse de molécules d'intérêt dans le domaine pharmaceutique ou agrochimique ainsi qu'en science des matériaux.

Les protocoles expérimentaux classiquement mis en oeuvre pour réaliser une amination réductrice sont :
- soit l'utilisation de quantité stoechiométriques de donneurs d'hydrures tels que les borohydrures (NaBH₄, NaBH₃CN ou NaBH(OAc)₃),
- soit l'hydrogénation catalytique.

L'utilisation de donneurs d'hydrures génère de nombreux déchets et les réactifs en eux-mêmes sont toxiques.

Dans le cas de l'hydrogénation catalytique, l'agent de réduction est l'hydrogène moléculaire, ce qui présente un intérêt écologique certain. La synthèse décrite dans le brevet EP 1 589 005 suit cette deuxième voie.

En effet, le brevet EP 1 589 005 décrit la synthèse du chlorhydrate de l'ivabradine à partir du composé de formule (II) : 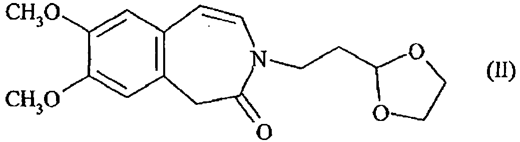 qui est soumis à une réaction d'hydrogénation catalytique en présence d'hydrogène et d'un catalyseur au palladium pour conduire au composé de formule (III) : 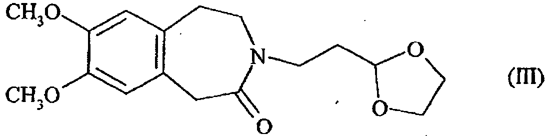 qui, sans être isolé, est mis en réaction, en présence d'hydrogène et d'un catalyseur au palladium, avec le composé de formule (IV) : 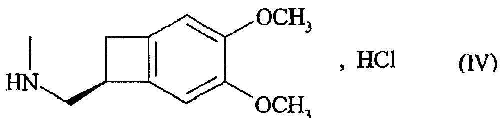 pour conduire à l'ivabradine de formule (I), sous forme de chlorhydrate.

L'inconvénient de cette voie de synthèse est d'utiliser un catalyseur au palladium.

Le palladium, tout comme le rhodium, le ruthénium ou l'iridium, métaux également utilisés pour catalyser les réactions d'amination réductrice, est un métal précieux dont la disponibilité limitée, et par conséquent le prix élevé, ainsi que la toxicité limitent l'intérêt.

Le brevet européen 2 202 225 décrit un procédé de synthèse de l'ivabradine via une réaction d'amination réductrice entre le composé de formule (VII) : 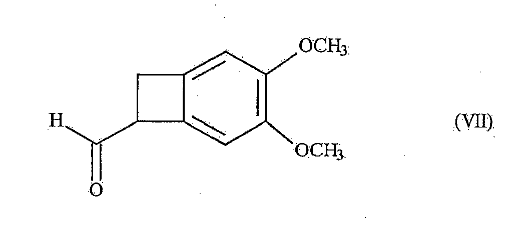 et le composé de formule (VIII) : 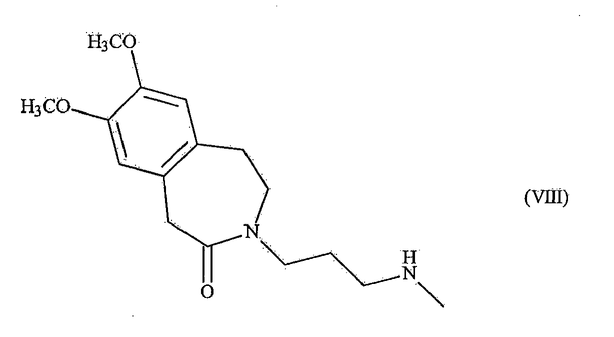 suivie par une étape de dédoublement du composé obtenu pour conduire à l'ivabradine.

La présente demande décrit une voie de synthèse de l'ivabradine qui permet de s'affranchir de l'utilisation d'un borohydrure ou d'un métal précieux.

La présente invention concerne un procédé de synthèse de l'ivabradine de formule (I): 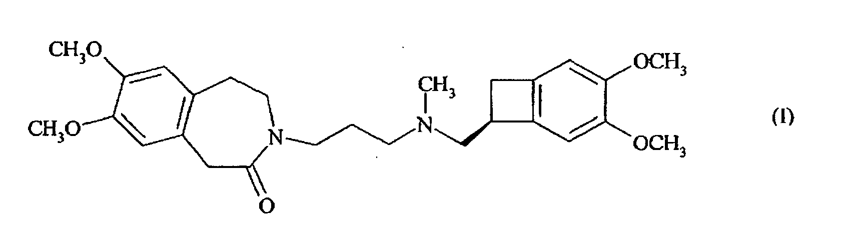 **caractérisé en ce que** l'on soumet le composé de formule (V) : 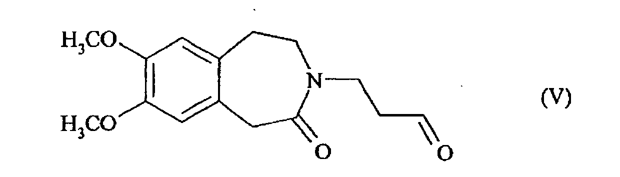 à une réaction d'amination réductrice par le composé de formule (VI) : 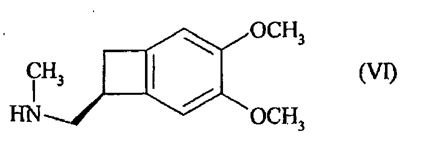 en présence de triéthylamine et d'acide formique,
en l'absence de solvant ou dans un solvant alcoolique.

L'utilisation de l'acide formique comme agent de réduction (réaction de Leuckart-Wallach) requiert parfois des températures de réaction très élevées, qui peuvent atteindre 180°C, et la formation secondaire de dérivés de type *N*-formyles est souvent observée.

La quantité d'acide formique engagée dans la réaction d'amination réductrice du composé de formule (V) par le composé de formule (VI) est supérieure à 1 équivalent par équivalent d'aldéhyde, plus préférentiellement comprise entre 2 et 50 équivalents par équivalent d'aldéhyde.

La quantité de triéthylamine engagée dans la réaction d'amination réductrice du composé de formule (V) par le composé de formule (VI) est supérieure à 1 équivalent par équivalent d'aldéhyde, plus préférentiellement comprise entre 2 et 50 équivalents par équivalent d'aldéhyde.

La température de la réaction d'amination réductrice entre le composé de formule (V) et le composé de formule (VI) est préférentiellement comprise entre 15 et 100°C, plus préférentiellement entre 30 et 100°C.

Parmi les solvants alcooliques qui peuvent éventuellement être utilisés pour effectuer la réaction d'amination réductrice du composé de formule (V) par le composé de formule (VI) on peut citer à titre non limitatif l'éthanol, l'isopropanol ou le trifluoroéthanol.

L'exemple ci-dessous illustre l'invention.

Les purifications par chromatographie sur colonne sont effectuées sur gel de silice 70-230 mesh.

Les spectres RMN ¹H sont enregistrés à 400 MHz.

Les déplacements chimiques sont exprimés en ppm (référence interne TMS).

Les abréviations suivantes ont été utilisées pour qualifier les pics : singulet (s), doublet (d), doublet de doublet (dd), triplet (t), quadruplet (q), multiplet (m).

### EXEMPLE 1 : 3-{3-[{[(7S)-3,4-diméthoxybicyclo[4.2.0]octa-1,3,5-trién-7-yl]méthyl}(méthyl)aminojpropyl}-7,8-diméthoxy-1,3,4,5-tétrahydro-2H-3-benzazépin-2-one

Dans un tube de Schlenk propre et sec, 0,25 mmol de 3-(7,8-diméthoxy-2-oxo-1,2,4,5-tétrahydro-3*H-*3-benzazépin-3-yl)propanal, 0,25 mmol de [(7*S*)-3,4-diméthoxybicyclo[4.2.0]octa-1,3,5-trién-7-yl]-*N*-méthylméthanamine et 1 mL (7,4 mmol) de triéthylamine sont agités à température ambiante sous atmosphere d'argon pendant une heure. 113 µL (3 mmol) d'acide formique sont ajoutés avec précaution et le mélange est chauffé à 85°C pendant 18h. Après refroidissement à température ambiante, le mélange réactionnel est dilué dans 5 mL d'une solution aqueuse de soude 3M. La phase aqueuse est extraite par trois fois 5 mL d'acétate d'éthyle. Les phases organiques sont réunies, lavées par une solution aqueuse saturée en NaCl (10 mL), séchées sur MgSO₄ et évaporées sous pression réduite.

Le produit brut est purifié sur gel de silice (éluant : pentane/acétate d'éthyle (95/5)) pour obtenir le produit attendu.
Rendement = 62 %
RMN ¹H (CDCl₃) : δ = 6,67 et 6,64 (2s, 2H) ; 6,55 et 6,50 (2s, 2H) ; 3,79 et 3,78 (2s, 12H) ; 3,76 (s, 2H) ; 3, 67 (m, 2H) ; 3,45 (m, 3H) ; 3,17 (dd, 1H) ; 2,99 (m, 2H) ; 2,65 (m, 2H) ; 2,50 (dd, 1H) ; 2,37 (t, 2H) ; 2,26 (s, 3H) ; 1,72 (q, 2H).

## Revendications

1. Procédé de synthèse de l'ivabradine de formule (I) : 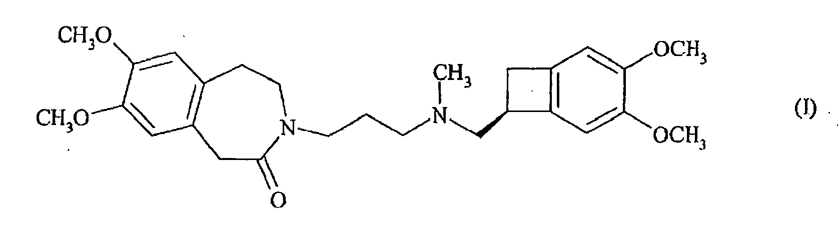 **caractérisé en ce que** l'on soumet le composé de formule (V) : 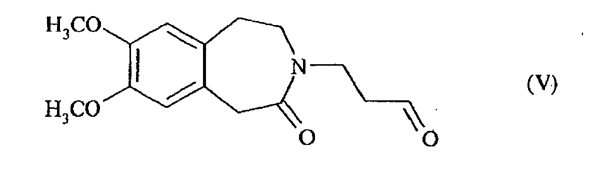 à une réaction d'amination réductrice par le composé de formule (VI) : 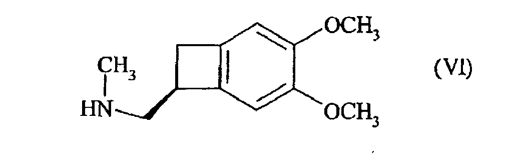 en présence d'acide formique en quantité supérieure à 1 équivalent par équivalent d'aldéhyde,
et de triéthylamine en quantité supérieure à 1 équivalent par équivalent d'aldéhyde,
à une température comprise entre 15 et 100°C,
en l'absence de solvant ou dans un solvant alcoolique.

2. Procédé de synthèse selon la revendication 1, **caractérisé en ce que** la réaction d'amination réductrice est conduite en l'absence de solvant.

3. Procédé de synthèse selon l'une des revendications 1 ou 2, **caractérisé en ce que** la quantité d'acide formique engagée dans la réaction d'amination réductrice est comprise entre 2 et 50 équivalents par équivalent d'aldéhyde.

4. Procédé de synthèse selon l'une des revendications 1 à 3, **caractérisé en ce que** la quantité de triéthylamine engagée dans la réaction d'amination réductrice est comprise entre 2 et 50 équivalents par équivalents d'aldéhyde.

5. Procédé de synthèse selon l'une des revendications 1 à 4, **caractérisé en ce que** la température de la réaction d'amination réductrice est comprise entre 30 et 100°C.

## Patentansprüche

1. Verfahren zur Synthese von Ivabradin der Formel (I): 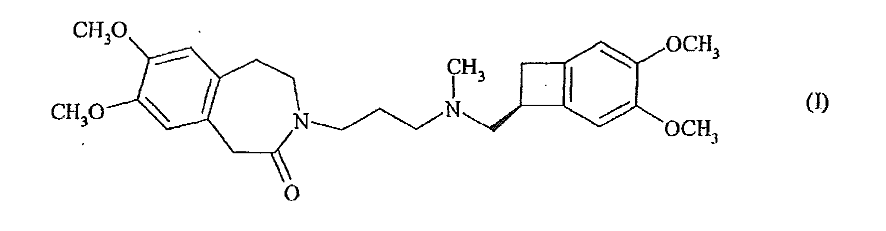 **dadurch gekennzeichnet, dass** man die Verbindung der Formel (V): 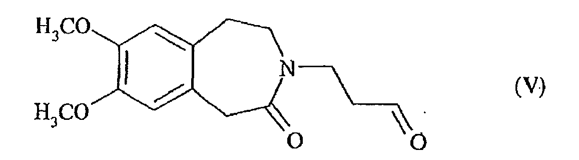 einer reduzierenden Aminierungsreaktion mit der Verbindung der Formel (VI): 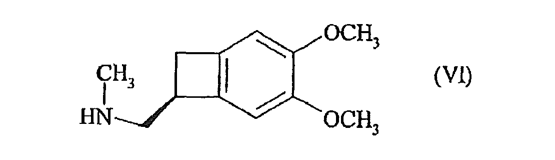 in Gegenwart von Ameisensäure in einer Menge von mehr als 1 Äquivalent pro Äquivalent des Aldehyds,
und von Triethylamin in einer Menge von mehr als 1 Äquivalent pro Äquivalent des Aldehyds,
bei einer Temperatur zwischen 15 und 100 °C
und in Abwesenheit eines Lösungsmittels oder in einem alkoholischen Lösungsmittel unterwirft.

2. Syntheseverfahren nach Anspruch 1, **dadurch gekennzeichnet, dass** die reduzierende Aminierungsreaktion in Abwesenheit eines Lösungsmittels durchgeführt wird.

3. Syntheseverfahren nach einem der Ansprüche 1 oder 2, **dadurch gekennzeichnet, dass** die bei der reduzierenden Aminierungsreaktion eingesetzte Menge der Ameisensäure zwischen 2 und 50 Äquivalenten pro Äquivalent des Aldehyds beträgt.

4. Syntheseverfahren nach einem der Ansprüche 1 bis 3, **dadurch gekennzeichnet, dass** die bei der reduzierenden Aminierungsreaktion eingesetzte Menge Triethylamin zwischen 2 und 50 Äquivalenten pro Äquivalent des Aldehyds beträgt.

5. Syntheseverfahren nach einem der Ansprüche 1 bis 4, **dadurch gekennzeichnet, dass** die Temperatur der reduzierenden Aminierungsreaktion zwischen 30 und 100 °C beträgt.

## Claims

1. Process for the synthesis of ivabradine of formula (I): 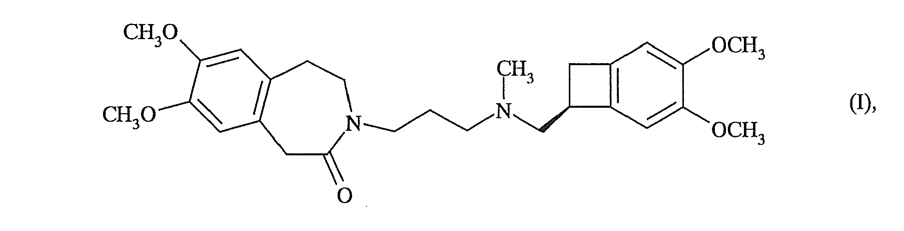 **characterised in that** the compound of formula (V): 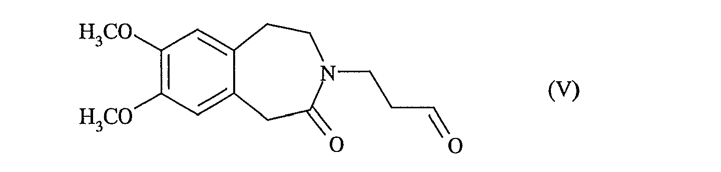 is subjected to a reductive amination reaction with the amine of formula (VI): 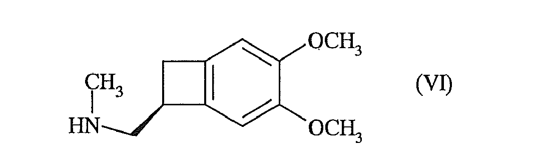 in the presence of formic acid in an amount greater than 1 equivalent per equivalent of aldehyde
and of triethylamine in an amount greater than 1 equivalent per equivalent of aldehyde,
at a temperature from 15 to 100°C,
in the absence of solvent or in an alcoholic solvent.

2. Synthesis process according to claim 1, **characterised in that** the reductive amination reaction is carried out in the absence of solvent.

3. Synthesis process according to one of claims 1 or 2, **characterised in that** the amount of formic acid used in the reductive amination reaction is from 2 to 50 equivalents per equivalent of aldehyde.

4. Synthesis process according to one of claims 1 to 3, **characterised in that** the amount of triethylamine used in the reductive amination reaction is from 2 to 50 equivalents per equivalent of aldehyde.

5. Synthesis process according to one of claims 1 to 4, **characterised in that** the temperature of the reductive amination reaction is from 30 to 100°C.
